# EUROPÄISCHE PATENTSCHRIFT

(11) **EP 0 548 650 B1**
(45) Veröffentlichungstag und Bekanntmachung des Hinweises auf die Patenterteilung: **01.04.1998**
(21) Anmeldenummer: 92120821.1
(22) Anmeldetag: 07.12.1992
(51) Int. Cl.: C07D 239/42, C07D 213/74, C07D 285/08, C07C 229/30, C07C 227/16, C07C 67/343

(54) **Verfahren zur Herstellung von substituierten 3-Alkoxy-acrylestern**
Process for the preparation of substituted 3-alkoxy-acrylic acid esters
Procédé de préparation de 3-alkoxy-acrylesters substitués

(30) Priorität: 20.12.1991 DE 4142191
(43) Veröffentlichungstag der Anmeldung: 30.06.1993
(73) Patentinhaber: BAYER AG, 51368 Leverkusen (DE)
(72) Erfinder: Kraus, Helmut, Dr., W-5000 Köln 80 (DE); Klausener, Alexander, Dr., W-5190 Stolberg (DE); Diehr, Hans-Joachim, Dr., W-5600 Wuppertal-Vohwinkel (DE)

(56) Entgegenhaltungen:
- EP-A- 0 383 117
- EP-A- 0 389 901

## Beschreibung

Die Erfindung betrifft ein Verfahren zur Herstellung von substituierten 3-Alkoxyacrylestern, welche als Fungizide oder als Zwischenprodukte zur Herstellung von fungiziden Acrylestern verwendet werden können.

Es ist bekannt, daß man bestimmte fungizid wirksame Alkoxyacrylester erhält, wenn man geeignete Essigsäureesterderivate mit Ameisensäureesterderivaten in Gegenwart einer Base kondensiert und dann die so erhältlichen 3-Hydroxyacrylesterderivate in einer zweiten Stufe oder gegebenenfalls auch in einer Eintopfreaktion, direkt der Kondensation folgend, mit Alkylierungsmitteln in Gegenwart einer Base an der 3-Hydroxygruppe alkyliert (vgl. z.B. DE-OS 3805059; DE-OS 3807232).

Der Nachteil dieses Verfahrens besteht darin, daß die als erste Stufe durchgeführte Claisen-Kondensation häufig nur sehr schlechte Ausbeuten liefert und bei manchen Essigsäureesterderivaten in Abhängigkeit von der Art des zur Alkoxycarbonylgruppe α-ständige Substituenten ganz versagt. So ist es beispielsweise in einigen Fällen ganz besonders schwierig, [(N-Pyridyl-N-alkyl)-amino]-essigsäurealkylester oder [(N-Pyrimidyl-N-alkyl)-amino]-essigsäurealkylester auf die genannte Weise überhaupt zur Reaktion zu bringen. Entweder es bedarf dazu der Verwendung starker Basen wie beispielsweise Lithiumdiisopropylamid, Kaliumhydrid oder Kalium-tert.-butanolat, was insbesondere unter technischer Aspekten wegen der damit verbundenen Sicherheitsprobleme unvorteilhaft und aufwendig ist, oder man überführt die Essigsäureesterderivate zunächst durch Umsetzung mit Orthoameisensäurediamidestern in substituierte 3-Amino-acrylester, die dann zu 3-Hydroxy acrylestern hydrolysiert und schließlich unter Verwendung basischer Hilfsmittel und geeigneter Alkylierungsmittel in die gewünschten 3-Alkoxy- acrylester überführt werden. Dieses Verfahren liefert im allgemeinen gute Ausbeuten, ist jedoch wegen der Verwendung des Orthoameisensäurediamidesters sowie wegen der Erforderlichkeit einer zusätzlichen Reaktionsstufe technisch aufwendig und wirtschaftlich unvorteilhaft.

Weiterhin ist bekannt, daß man fungizid wirksame substituierte 3-Alkoxyacrylester erhält, wenn man geeignete Essigsäureesterderivate mit Formamiden oder Formamidderivaten umsetzt, die dabei anfallenden substituierten 3-Amino-acrylester zu 3-Hydroxy-acrylestern hydrolisiert und schließlich unter Verwendung basischer Hilfsmitel in die gewünschten 3-Alkoxy-acrylester überführt (z.B. DE-OS 3 807 232; DE-OS 3 910 358).

Die dabei verwendeten Dimethylformamiddialkylacetale liefern jedoch häufig nur sehr geringe Ausbeuten an gewünschtem Zwischenprodukt 3-Amino-acrylester.

Es bestand daher die Aufgabe, ein Verfahren zu finden, das die Synthese der gewünschten 3-Alkoxy-acrylester, ausgehend von Essigsäureester-derivaten, unter Zuhilfenahme technisch einfacher Mittel und gut zugänglicher und handhabbarer-Ausgangsmaterialien erlaubt.

Es wurde nun gefunden, daß man substituierte 3-Alkoxy-acrylester der allgemeinen Formel (I), 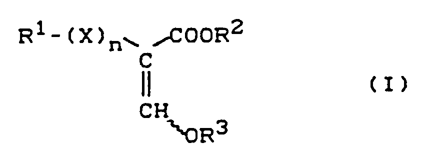 in welcher
- R¹: für einen gegebenenfalls einfach oder mehrfach, gleich oder verschieden substituierten, gesättigten, einfach oder mehrfach ungesättigten oder aromatischen Carbocyclus mit 5 bis 10 Kohlenstoffatomen oder für einen gegebenenfalls einfach oder mehrfach, gleich oder verschieden substituierten, gesättigten, einfach oder mehrfach ungesättigten oder aromatischen Heterocyclus mit 2 bis 9 Kohlenstoffatomen und 1 bis 5 gleichen oder verschiedenen Heteroatomen - insbesondere Stickstoff, Sauerstoff oder Schwefel - steht, wobei als Substituenten jeweils infrage kommen:
Halogen, Cyano, Nitro, jeweils geradkettiges oder verzweigtes Alkyl, Alkoxy oder Alkylthio mit jeweils 1 bis 6 Kohlenstoffatomen, jeweils geradkettiges oder verzweigtes Halogenalkyl, Halogenalkoxy oder Halogenalkylthio mit jeweils 1 bis 4 Kohlenstoffatomen und 1 bis 9 gleichen oder verschiedenen Halogenatomen, jeweils geradkettiges oder verzweigtes Alkoxycarbonyl oder Alkoximinoalkyl, Dialkylamino oder Dialkylaminocarbonyl mit jeweils 1 bis 4 Kohlenstoffatomen in den einzelnen Alkylteilen, jeweils geradkettiges oder verzweigtes Alkenyl oder Alkinyl mit jeweils 2 bis 8 Kohlenstoffatomen, Cycloalkyl mit 3 bis 7 Kohlenstoffatomen, zweifach verknüpftes Alkandiyl mit 3 bis 5 Kohlenstoffatomen, oder jeweils im Arylteil oder im Heteroarylteil gegebenenfalls einfach oder mehrfach, gleich oder verschieden durch Halogen, Alkyl, Alkoxy, Alkylthio, Halogenalkyl, Halogenalkoxy, Halogenalkylthio, Dioxyalkylen, Halogen- substituiertes Dioxyalkylen oder gegebenenfalls substituiertes Phenyl substituiertes Aryl, Aryloxy, Arylthio, Arylcarbonyl, Aralkyl Aralkenyl, Aralkinyl, Aralkyloxy, Aralkylthio, Heteroarylalkyl, Heteroarylalkenyl, Heteroyryloxy, Heteroarylthio, Heteroarylcarbonyl, Heteroarylalkyloxy, Heteroarylalkylthio, oder Heteroaryl mit jeweils 6 bis 10 Kohlenstoffatomen im Arylteil oder mit 2 bis 9 Kohlenstoffatomen und 1 bis 4 gleichen oder verschiedenen Heteroatomen - insbesondere Stickstoff, Sauerstoff und/oder Schwefel - im Heteroarylteil und gegebenenfalls 1 bis 6 Kohlenstoffatomen im geradkettigen oder verzweigten Alkylteil oder gegebenenfalls 2 bis 6 Kohlenstoffatomen im geradkettigen oder verzweigten Alkenylteil beziehungsweise Alkinylteil;
- R²: für geradkettiges oder verzweigtes Alkyl mit 1 bis 6 Kohlenstoffatomen oder für gegebenenfalls im Arylteil einfach oder mehrfach, gleich oder verschieden substituiertes Aralkyl mit 1 bis 4 Kohlenstoffatomen im geradkettigen oder verzweigten Alkylteil und 6 bis 10 Kohlenstoffatomen im Arylteil steht, wobei als Arylsubstituenten infrage kommen: Halogen, Cyano, Nitro, jeweils geradkettiges oder verzweigtes Alkyl, Alkoxy oder Alkylthio mit jeweils 1 bis 4 Kohlenstoffatomen, jeweils geradkettiges oder verzweigtes Halogenalkyl, Halogenalkoxy oder Halogenalkylthio mit jeweils 1 bis 4 Kohlenstoffatomen und 1 bis 9 gleichen oder verschiedenen Halogenatomen, jeweils geradkettiges oder verzweigtes Alkoxycarbonyl oder Alkoximinoalkyl mit jeweils 1 bis 8 Kohlenstoffatomen in den einzelnen Alkylteilen, Cycloalkyl mit 3 bis 7 Kohlenstoffatomen, zweifach verknüpftes Alkandiyl mit 3 bis 5 Kohlenstoffatomen, jeweils gegebenenfalls im Arylteil einfach oder mehrfach, gleich oder verschieden durch Halogen, Alkyl, Alkoxy, Alkylthio, Halogenalkyl, Halogenalkoxy oder Halogenalkylthio mit jeweils 1 bis 4 Kohlenstoffatomen und gegebenenfalls 1 bis 9 gleichen oder verschiedenen Halogenatomen substituiertes Aryl, Aralkyl, Aryloxy oder Aralkyloxy mit jeweils 6 bis 10 Kohlenstoffatomen im Arylteil und gegebenenfalls 1 bis 4 Kohlenstoffatomen im geradkettigen oder verzweigten Alkylteil oder jeweils gegebenenfalls im Heteroarylteil einfach oder mehrfach, gleich oder verschieden durch Halogen, Alkyl, Alkoxy, Alkylthio, Halogenalkyl, Halogenalkoxy oder Halogenalkylthio mit jeweils 1 bis 4 Kohlenstoffatomen und gegebenenfalls 1 bis 9 gleichen oder verschiedenen Halogenatomen substituiertes Heteroarylalkyl oder Heteroaryl mit jeweils 2 bis 9 Kohlenstoffatomen und 1 bis 4 gleichen oder verschiedenen Heteroatomen, - insbesondere Stickstoff, Sauerstoff und/oder Schwefel - im Heteroarylteil und gegebenenfalls 1 bis 4 Kohlenstoffatomen im geradkettigen oder verzweigten Alkylteil;
- R³: für geradkettiges oder verzweigtes Alkyl mit 1 bis 6 Kohlenstoffatomen oder für gegbenenfalls im Acrylteil einfach oder mehrfach, gleich oder verschieden substituiertes Aralkyl mit 1 bis 4 Kohlenstoffatomen im geradkettigen oder verzeigten Alkylteil und 6 bis 10 Kohlenstoffatomen im Arylteil steht, wobei als Arylsubstituenten infrage kommen: Halogen, Cyano; Nitro, jeweils geradkettiges oder verzweigtes Alkyl, Alkoxy oder Alkylthio mit jeweils 1 bis 4 Kohlenstoffatomen, jeweils geradkettiges oder verzweigtes Halogenalkyl, Halogenalkoxy oder Halogenalkylthio mit jeweils 1 bis 4 Kohlenstoffatomen und 1 bis 9 gleichen oder verschiedenen Halogenatomen, jeweils geradkettiges oder verzweigtes Alkoxycarbonyl oder Alkoximinoalkyl mit jeweils 1 bis 8 Kohlenstoffatomen in den einzelnen Alkylteilen, Cycloalkyl mit 3 bis 7 Kohlenstoffatomen, zweifach verknüpftes Alkandiyl mit 3 bis 5 Kohlenstoffatomen, jeweils gegebenenfalls im Arylteil einfach oder mehrfach, gleich oder verschieden durch Halogen, Alkyl, Alkoxy, Alkylthio, Halogenalkyl, Halogenalkoxy oder Halogenalkylthio mit jeweils 1 bis 4 Kohlenstoffatomen und gegebenenfalls 1 bis 9 gleichen oder verschiedenen Halogenatomen substituiertes Aryl, Aralkyl, Aryloxy oder Aralkyloxy mit jeweils 6 bis 10 Kohlenstoffatomen im Arylteil und gegebenenfalls 1 bis 4 Kohlenstoffatomen im geradkettigen oder verzweigten Alkylteil oder jeweils gegebenenfalls im Heteroarylteil einfach oder mehrfach, gleich oder verschieden durch Halogen, Alkyl, Alkoxy, Alkylthio, Halogenalkyl, Halogenalkoxy oder Halogenalkylthio mit jeweils 1 bis 4 Kohlenstoffatomen und gegebenenfalls 1 bis 9 gleichen oder verschiedenen Halogenatomen substitu-iertes Heteroarylalkyl oder Heteroaryl mit jeweils 2 bis 9 Kohlenstoffatomen und 1 bis 4 gleichen oder verschiedenen Heteroatomen, - insbesondere Stickstoff, Sauerstoff und/oder Schwefel - im Heteroarylteil und gegebenenfalls 1 bis 4 Kohlenstoffatomen im geradkettigen oder verzweigten Alkylteil;
- X: für Sauerstoff, Schwefel oder für einen der Reste 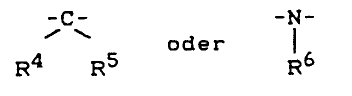 steht und
- n: für eine Zahl 0 oder 1 steht, wobei
R⁴, R⁵ und R⁶ unabhängig voneinander jeweils für Wasserstoff, für jeweils geradkettiges oder verzweigtes Alkyl mit 1 bis 6 Kohlenstoffatomen, Alkenyl mit 2 bis 6 Kohlenstoffatomen oder für jeweils gegebenenfalls im Arylteil einfach oder mehrfach, gleich oder verschieden substituiertes Aralkyl mit 1 bis 6 Kohlenstoffatomen im geradkettigen oder verzweigten Alkylteil und 6 bis 10 Kohlenstoffatomen im Arylteil oder Aralkenyl mit 2 bis 6 Kohlenstoffatomen im geradkettigen oder verzweigten Alkenylteil und 6 bis 10 Kohlenstoffatomen im Arylteil stehen, wobei als Arylsubstituenten jeweils die bei R² genannten infrage kommen.
erhält, wenn man substituierte Essigsäureester der Formel (II),

R¹-(X)ₙ-CH₂-COOR² (II)

in welcher
R¹, R², X und n die oben angegebene Bedeutung haben, in Gegenwart eines geeigneten Verdünnungsmittels zunächst mit Kohlenmonoxid bei einem Kohlenmonoxid-Partialdruck von 1 bis 200 bar und einem basischen Hilfsmittel umsetzt, und dabei Zwischenprodukte der allgemeinen Formel (III), 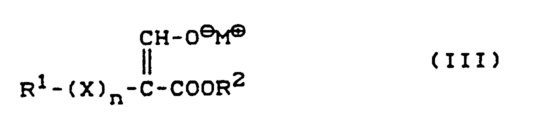 in welcher
- R¹, R², X und n: die oben angegebene Bedeutung haben und
- M^{⊕}: für Wasserstoff oder das Äquivalent eines Alkalimetall- oder Erdalkalimetall- oder Ammoniumkations (vorzugsweise eines Natrium- oder Kaliumkations) steht,
erhält, wonach aus diesen Salzen die freien Enole durch Ansäuern mit verdünnten Säuren, wie beispielsweise verdünnte Salzsäure und darauffolgende Extraktion mit einem geeigneten organischen Lösungsmittel wie beispielsweise Essigester isoliert werden und dann in einem geeigneten Verdünnungsmittel in Gegenwart einer geeigneten Base mit dem Alkylierungsmittel bei Temperaturen von 20° C bis 60° C alkyliert werden.

Alternativ ist es möglich, die Enolatsalze der Formel (III) direkt im Reaktionsgemisch mit dem Alkylierungsmittel umzusetzen, In einer besonders bevorzugten Variante dieser "Eintopfreaktion" arbeitet man zunächst im Verlauf der Carbonylierung mit einem Überschuß des basischen Hilfsmittels, beispielsweise Natriummethylat; man erhält dadurch in der Reaktionsmischung das Enolat der Formel (III) in Form des Natriumsalzes sowie nicht umgesetztes Natriummethylat. Dieses nicht umgesetzte Natriummethylat läßt sich mit einer geeigneten Säure, wie beispielsweise Methansulfonsäure, neutralisieren, darauf gibt man zusammen mit einem weiteren Äquivalent Base, wie beispielsweise Kaliumcarbonat, das Methylierungsmittel zu.

Durch diese Vorgehensweise erübrigt es sich, das Methylierungsmittel im Überschuß einzusetzen, was sonst erforderlich wäre, um Verluste durch seine Reaktion mit Natriummethylat auszugleichen.

In einer anderen besonders bevorzugten Variante dieser "Eintopfreaktion" arbeitet man im Verlaufe der Carbonylierung zunächst mit einer stöchiometrischen Menge oder einem geringfügigen (etwa 5 bis 35%iger) Überschuß des basischen Hilfsmittels, beispielsweise Natriummethylat.

Man erhält dadurch in der Reaktionsmischung das Enolat der Formel (III) in Form des Natriumsalzes sowie nunmehr nur noch sehr geringe, aber dem eingesetzten Überschuß des basischen Hilfsmittel entsprechende Mengen unumgesetztes Natriummethylat. Insbesondere, wenn die Begrenzung des auf zuwendenden Natriummethylats auf die stöchiometrisch erforderliche Menge gelingt, erübrigt sich ein Abfangen von nicht umgesetztem basischen Hilfsmittel, etwa durch Zusatz einer geeigneten Säure, und das Alkylierungsmittel kann direkt in das Reaktionsgemisch eingetragen werden. Diese Vorgehensweise erlaubt es, die Verwendung von Überschüssen an Alkylierungsreagenz einzugrenzen und in günstig gelagerten Fällen ganz zu vermeiden.

Die so erhältichen Alkoxymethylencarbonester der Formel (I) sind ebenso wie die Enole beziehungsweise Enolsalze der Formel (III) im Prinzip bekannt (vergl. EP 384 211, EP 331 966, EP 383 117) und potente Fungizide.

Es ist ausgesprochen überraschend, daß die erfindungsgemäße Umsetzung der Essigsäureester-derivate der allgemeinen Formel (II) mit Kohlenmonoxid auch in solchen Fallen glatt gelingt, in denen die sehr ähnliche Umsetzung des Ausgangsmaterials mit Ameisensäuremethylester in Gegenwart von Basen oder die Umsetzung mit Dialkylformamiddialkylacetalen gänzlich oder fast völlig versagen und auch die Umsetzung mit Orthoameisensäurediamidestern, die allerdings sehr viel aufwendiger ist, nur mit unzureichendem Erfolg abläuft.

Daher ist es ein besonderer Vorteil des erfindungsgemäßen Verfahrens, daß mit seiner Anwendung Verbindungen zugänglich sind, die vorher garnicht oder nur in sehr geringer Ausbeute zu erhalten waren, und zwar mit einer gegenüber dem bekannten Stand der Technik vereinfachten Vorgehensweise, die sich inbesondere für das Arbeiten in technischen Apparaturen eignet.

Ein weiterer Vorteil des erfindungsgemäßen Verfahrens besteht darin, daß sich die mit Hilfe des erfindungsgemäßen Verfahrens einfach und in hoher Ausbeute erhältlichen substituierten Enole beziehungsweise Enolsalze der allgemeinen Formel (III) hervorragend zur Herstellung von fungiziden 3-Alkoxy-acrylestern oder auch anderen 3-substituierten Acrylestern eignen, wenn man sie mit
Hilfe einfacher bekannter Verfahren entweder direkt in einer zweiten separaten Reaktionsstufe oder direkt anschließend im Eintopfverfahren alkyliert oder mit Hilfe Hilfe einfacher bekannter Verfahren entweder direkt in einer zweiten separaten Reaktionsstufe oder direkt anschließend im Eintopfverfahren alkyliert oder mit Hilfe gleichfalls bekannter einfacher Verfahren für Enole oder Enolsalze typischen Folgereaktionen unterwirft, wobei exemplarisch, aber nicht im ausschließlichen Sinne genannt seien:
- Umsetzung mit Aminen zu substituierten 3-Amino-acryl säureestern,
- Umsetzung mit Mercaptanen zu substituierten 3-Alkylthio- oder 3-Arylthio-arcylsäureetern oder
- Umsetzung mit Säurehalogeniden zu 3-Acyloxy- oder 3-Sulfonyloxy-acrylsäureestern.

Besonders bevorzugt herstellbar sind Verbindungen der Formel (I), bei welchen
- R¹: für einen gegebenenfalls ein- bis fünffach, gleich oder verschieden substituierten und/oder benzannelierten Phenylrest oder für einen gegebenenfalls ein- bis fünffach, gleich oder verschieden substituierten Cycloalkenylrest mit 5 bis 7 Kohlenstoffatomen oder für einen gegebenenfalls ein- bis fünffach, gleich oder verschieden substituierten und/oder benzannelierten Heteroarylrest mit 1 bis 5 Kohlenstoffatomen und 1 bis 3 Heteroatomen - insbesondere Stickstoff, Sauerstoff oder Schwefel - steht, wobei als Substituenten jeweils infrage kommen:
Fluor, Chlor, Brom, Cyano, Nitro, Methyl, Ethyl, n- oder i-Propyl, n-, i-, s- oder t-Butyl, Methoxy, Ethoxy, n- oder i-Propoxy, Methylthio, Trifluormethyl, Trifluormethoxy, Trifluormethylthio, Methoxycarbonyl, Ethoxycarbonyl, Methoximinomethyl, Ethoximinomethyl, Methoximinoethyl, Ethoximinoethyl, Dimethylamino, Diethylamino, Dimethylcarbamoyl, Diethylcarbamoyl, Allyl, Butenyl oder Propargyl, Cyclopropyl, Cyclopentyl, Cyclohexyl, 1,3-Propandiyl, 1,4-Butandiyl, 1,5-Pentandiyl oder jeweils gegebenenfalls ein- bis dreifach, gleich oder verschieden durch Fluor, Chlor, Brom, Methyl, Ethyl, n- oder i-Propyl, n-, i-, s- oder t-Butyl, Methoxy, Ethoxy, n- oder i-Propoxy, Methylthio, Ethylthio, Trifluormethyl, Trifluormethoxy, Trifluormethylthio, Dioxymethylen oder gegebenenfalls ein- bis dreifach, gleich oder verschieden durch Fluor, Chlor, Methyl, Methoxy oder Trifluormethyl substituiertes Phenyl, substituiertes Phenyl, Naphthyl, Phenoxy, Phenylthio, Phenylcarbonyl, Benzyl, Phenylethyl, Phenylpropyl, Phenylethenyl, Benzyloxy, Heteroaryloxy, Heterarylmethyl oder Heteroaryl, wobei als Heteroarylreste im einzelnen jeweils die folgenden infrage kommen: 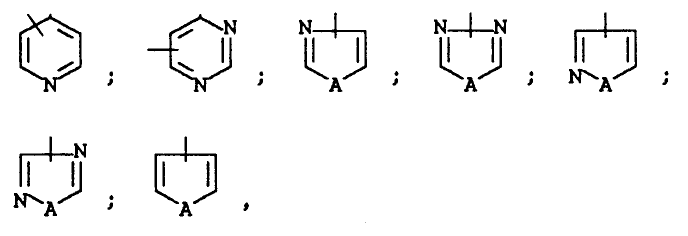 welche gegebenenfalls auch benzanneliert sein können und bei welchen
- A: jeweils für Sauerstoff, Schwefel, eine NH- oder NCH₃-Gruppe steht;
- R²: für Methyl, Ethyl, n- oder i-Propyl, n-, i-, s- oder t-Butyl oder für gegebenenfalls ein- bis dreifach, gleich oder verschieden substituiertes Benzyl steht, wobei als Substituenten infrage kommen:
Fluor, Chlor, Brom, Cyano, Nitro, Methyl, Ethyl, n- oder i-Propyl, n-, i-, s- oder t-Butyl, Methoxy, Ethoxy, n- oder i-Propoxy, Methylthio, Trifluormethyl, Trifluormethoxy, Difluormethoxy, Trifluormethylthio, Methoxycarbonyl, Ethoxycarbonyl, Methoximinomethyl, Ethoximinomethyl, Methoximinoethyl, Ethoximinoethyl, Cyclopentyl, Cyclohexyl, 1,3-Propandiyl, 1,4-Butandiyl oder jeweils gegebenenfalls im Phenylteil ein- bis dreifach, gleich oder verschieden durch Fluor, Chlor, Brom, Methyl, Ethyl, Methoxy, Ethoxy, Methylthio, Trifluormethyl, Difluormethoxy, Trifluormethoxy und/oder Trifluormethylthio, substituiertes Phenyl, Benzyl, Phenoxy oder Benzyloxy,
- R³: für Methyl, Ethyl, n- oder i-Propyl, n-, i-, s- oder t-Butyl steht,
- X: für Sauerstoff, Schwefel oder für einen der Reste 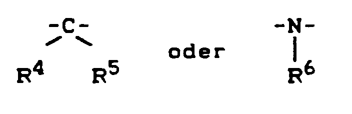 steht und
- n: für eine Zahl 0 oder 1 steht, wobei
- R⁴, R⁵ und R⁶: unabhängig voneinander jeweils für Wasserstoff, Methyl, Ethyl, n- oder i-Propyl, n-, i-, s- oder t-Butyl oder für jeweils gegebenenfalls ein- bis dreifach, gleich oder verschieden substituiertes Benzyl oder Phenyl steht, wobei als Substituenten jeweils die bei R² genannten infrage kommen.

Ganz besonders bevorzugt herstellbar sind Verbindungen der allgemeinen Formel (I), bei welchen
- R¹: für einen gegebenenfalls ein- bis dreifach substuierten und/oder benzannelierten Phenylrest, für einen jeweils gegebenenfalls ein- bis dreifach, gleich oder verschieden substituierten und/oder benzannelierten Cyclohexenylrest oder Cyclopentenylrest oder für einen gegebenenfalls ein- bis dreifach substituierten und/oder benzannelierten Heteroarylrest steht, wobei als Heteroarylreste insbesondere infrage kommen: 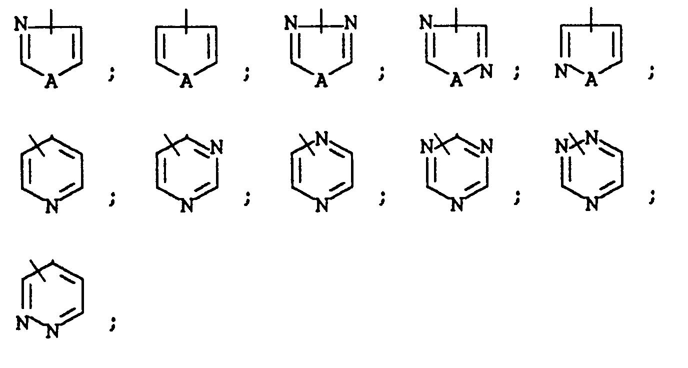 wobei
- A: jeweils für Sauerstoff, Schwefel, eine NH- oder NCH₃-Gruppe steht und wobei als Phenyl-, Cyclopentenyl-, Cyclohexenyl- oder Heteroarylsubstituenten jeweils infrage kommen:
Fluor, Chlor, Brom, Cyano, Nitro, Methyl, Ethyl, Methoxy, Methylthio, Trifluormethyl, Trifluormethoxy, Trifluormethylthio, Methoxycarbonyl, Ethoxycarbonyl oder Dimethylamino oder jeweils gegebenenfalls ein- bis dreifach, gleich oder verschieden durch Fluor, Chlor, Methyl, Ethyl, Methoxy, Trifluormethyl, Dioxymethylen oder Phenyl, Phenoxy, Phenylcarbonyl, Benzyl, Pyridyl, Pyrazolyl, Oxazolyl, Thiazolyl, Thienyl, Furyl, Thiadiazolyl, Oxadiazolyl, Imidazolyl oder Triazolyl,
- R²: für Methyl, Ethyl, n- oder i-Propyl oder Benzyl steht,
- R³: für Methyl, Ethyl, n- oder i-Propyl oder Benzyl steht,
- X: für Sauerstoff, Schwefel oder für einen der Reste 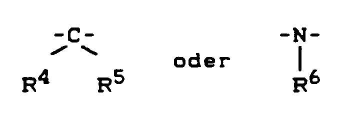 steht und
- n: für eine Zahl 0 oder 1 steht, wobei
- R⁴, R⁵ und R⁶: unabhängig voneinander jeweils für Wasserstoff, Methyl, Ethyl, n- oder i-Propyl oder Benzyl stehen.

Aryl als solches oder in Zusammensetzungen bedeutet vorzugsweise Phenyl oder Naphthyl, insbesondere Phenyl.

Alle aliphatischen Reste als solche oder in Zusammensetzungen sind geradkettig oder verzweigt.

Halogen steht, wenn nicht anders definiert, für Fluor, Chlor, Brom oder Iod, insbesondere für Fluor, Chlor oder Brom.

Verwendet man beispielsweise N-Methyl-N-[2-(6-phenyl)-pyridyl]-glycinmethylester als Ausgangsstoff, so läßt sich der Reaktionsablauf des erfindungsgemäßen Verfahrens durch das folgende Formelschema darstellen:

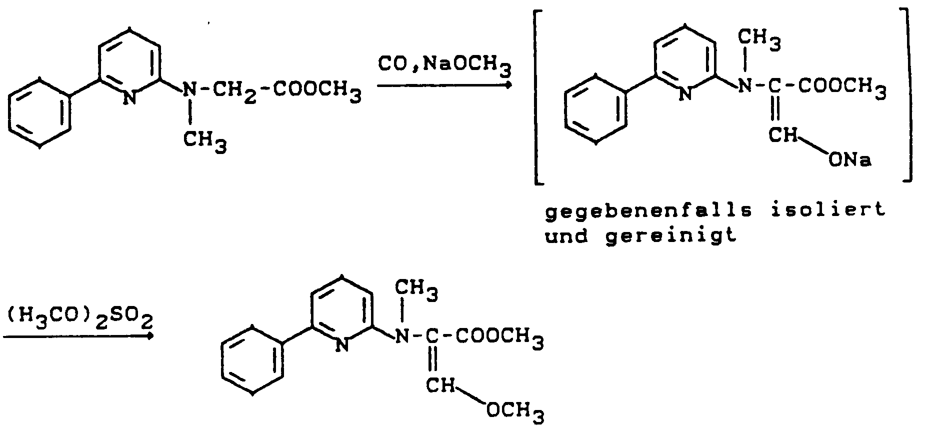

Die zur Durchführung des erfindungsgemäßen Verfahrens als Ausgangsstoffe benötigten substituierten Essigsäureester sind durch die Formel (II) allgemein definiert. In dieser Formel (II) stehen R¹, R², X und n vorzugsweise für diejenigen Reste, die bereits im Zusammenhang mit der Beschreibung der erfindungsgemäßen Stoffe der Formel (I) als bevorzugt für diese Substituenten genannt wurden.

Die substituierten Essigsäureester der Formel (II) sind bekannt oder erhältlich in Analogie zu bekannten Verfahren (vgl. z.B. J. Chem. Pharm. Bull. 23, 3008-3010 [1975]; Prakt. Chem. 315, 1175-1182 [1973]; Chimia 28, 235-236 [1974]; Pak. J. Sci. Ind. Res. 20, 139-149 [1977]; J. Heterocycl. Chem. 5, 281-283 [1968]; Pol. J. Chem. 53, 2349-2354 [1979]; J. Heterocycl. Chem. 24, 85-89 [1987]; Zh. org. Khim. 20, 1517-1538 [1984]; Zh. Org. Khim. 20, 2002-2011 [1984]; Izv. Akad. Nauk SSSR. Ser. Khim. 1984, 2760-2765; DE 21 03 728; DE 26 37 911; DE 27 09 108; DE 27 25 361; DE 24 25 282; GB 11 61 492 [1969]; EP 182 769; EP 245 230; EP 227 932; DE-OS 3904931; DE-OS 3805059; DE-OS 3807232; DE-OS 3905119; DE-OS 3904931).

Als basische Hilfsmittel kommen alle üblichen verwendbaren und unter den Reaktionsbedingungen aktiven anorganischen und organischen Basen in Frage. Vorzugsweise verwendet man Alkalimetallhydride, -hydroxide, -amide oder -alkoholate, wie beispielsweise Natriumhydrid, Natriumamid, Natriumhydroxid, Natriummethylat, Natriummethylat oder Kalium-t-butylat, besonders bevorzugt ist die Verwendung von Natriummethylat, Natriumethylat und K-t-butylat.

Als Verdünnungsmittel zur Durchführung der ersten Stufe beziehungsweise des ersten Teilschrittes des erfindungsgemäßen Verfahrens kommen vor allem inerte organische Lösungsmittel in Frage. Hierzu gehören insbesondere aliphatische, alicyclische oder aromatische, gegebenenfalls auch halogenierte Kohlenwasserstoffe wie beispielsweise Benzin, Benzol, Toluol, Xylol, Chlorbenzol, Petrolether, Hexan, Cyclohexan, Dichlormethan, Chloroform, Tetrachlorkohlenstoff, Ether wie Diethylether, Dioxan, Tetrahydrofuran oder Ethylenglykoldimethyl- oder -diethylether, Amide, wie Dimethylformamid, Dimethylacetamid, N-Methylformanilid, N-Methylpyrrolidon oder Hexamethylphosphorsäuretriamid.

Ferner können Alkohole der allgemeinen Formel R²-OH verwendet werden, wobei der Rest R² mit den in der allgemeinen Formel (I), (II) und (III) beschriebenen identisch sein sollte, um die Bildung von Produktgemischen durch unter den Reaktionsbedingungen mögliche Umesterungsreaktionen zu vermeiden.

Es lassen sich aber auch Gemische der genannten Lösungsmittel einsetzen.

Als Verdünnungsmittel für die Durchführung der zweiten Stufe beziehungsweise des zweiten Teilschrittes des erfindungsgemäßen Verfahrens kommen die gleichen Lösungsmittel wie die für die Durchführung der ersten Stufe beziehungsweise des ersten Teilschrittes in Frage. Dabei kann es nützlich sein, als Lösungsmittel für die zweite Stufe beziehungsweise den zweiten Teilschritt ein anderes als für die erste Stufe beziehungsweise den ersten Teilschritt zu verwenden.

Weiterhin kann es von Vorteil sein, nach Durchführung des ersten Teilschrittes beziehungsweise der ersten Stufe des erfindungsgemäßen Verfahrens das Reaktionsgemisch unter vermindertem oder erhöhtem Druck oder unter Normaldruck gegebenenfalls bis zur Trockne einzuengen, dann gegebenenfalls die Zwischenstufe der allgemeinen Formel (III), bevorzugt als Enol-Salz, beispielsweise durch Filtration unter vermindertem oder erhöhtem Druck oder unter Normaldruck zu isolieren und, gegebenenfalls nach Zwischenreinigung, beispielsweise durch Waschen oder Extrahieren mit einem geeigneten organischen Lösungsmittel, den so erhaltenen Feststoff in einem der oben beschriebenen Verdünnungsmittel zu lösen oder zu dispergieren und dann die zweite Stufe beziehungsweise den zweiten Teilschritt der erfindungsgemäßen Reaktion durchzuführen.

Ist das eingesetzte Alkylierungsmittel zur Durchführung der zweiten Stufe beziehungsweise des zweiten Teilschrittes des erfindungsgemäßen Verfahrens unter den Reaktionsbedingungen flüssig, so kann gegebenenfalls auf den Zusatz eines Verdünnungsmittels völlig verzichtet werden.

Die Reaktionstemperaturen können bei der Durchführung des erfindungsgemäßen Verfahrens in einem größeren Bereich variiert werden.

Im allgemeinen arbeitet man bei der Durchführung des ersten Teilschrittes beziehungsweise der ersten Stufe des erfindungsgemäßen Verfahrens bei Temperaturen zwischen 0°C und 200°C, bevorzugt 20°C und 150°C, und bei der Durchführung des zweiten Teilschrittes beziehungsweise der zweiten Stufe des erfindungsgemäßen Verfahrens bei Temperaturen zwischen -80°C und 100°C, bevorzugt - 40°C und 70°C.

Die erste Stufe beziehungsweise der erste Teilschritt des erfindungsgemäßen Verfahrens wird unter erhöhtem Druck durchgeführt.

Der CO-Partialdruck beträgt zwischen 1 bar und 200 bar, bevorzugt zwischen 5 bar und 150 bar, besonders bevorzugt zwischen 10 bar und 70 bar, Der Gesamtdruck beträgt zwischen 5 bar und 200 bar, bevorzugt zwischen 10 bar und 150 bar, besonders bevorzugt zwischen 10 bar und 70 bar. Der Differenzbetrag zwischen Gesamtdruck und CO-Partialdruck kann dabei durch Aufpressen eines Inertgases, z.B. Stickstoff oder Argon, aufgebracht werden.

Die zweite Stufe beziehungsweise der zweite Teilschritt des erfindungsgemäßen Verfahrens kann unter vermindertem oder erhöhtem Druck durchgeführt werden, bevorzugt arbeitet man jedoch unter Normaldruck.

Gegebenenfalls kann bei der Durchführung der zweiten Stufe beziehungsweise des zweiten Teilschritts des erfindungsgemäßen Verfahrens der Einsatz einer Inertgasatmosphäre wie beispielsweise Stickstoff oder Argon zweckmäßig sein, im allgemeinen ist es jedoch möglich, diesen Teilschritt beziehungsweise diese Stufe des erfindungsgemäßen Verfahrens unter normaler Raumluftatmosphäre durchzuführen.

Zur Durchführung des ersten Teilschritts beziehungsweise der ersten Stufe des erfindungsgemäßen Verfahrens setzt man pro Mol an substituierten Essigsäureestern der Formel (II) im allgemeinen 1,0 bis 15,0 Mol, vorzugsweise 1,0 bis 5,0 Mol an basischen Hilfsmitteln ein.

Die Reaktionsdurchführung, Aufarbeitung und gegebenenfalls die Isolierung der Reaktionsprodukte erfolgt nach allgemein üblichen Methoden (vergleiche auch die Herstellungsbeispiele).

Zur Durchführung des zweiten Teilschrittes beziehungsweise der zweiten Stufe des erfindungsgemäßen Verfahrens setzt man pro Mol an substituierten Essigsäureestern der allgemeinen Formel (II) beziehungsweise pro Mol zwischen islierten oder auch nicht zwischenisolierten Enols der allgemeinen Formel (III) 1,0 bis 10,0 Mol, bevorzugt 1,0 bis 2,0 Mol an basischen Hilfsmitteln und 1,0 bis 20,0 Mol, bevorzugt 1,0 bis 5,0 Mol an Alkylierungsreagenz ein.

Die Reaktionsführung, Aufarbeitung und Islierung der Reaktionsprodukte erfolgt nach allgemein üblichen Methoden (vergleiche auch die Herstellungsbeispiele).

### Beispiel 1

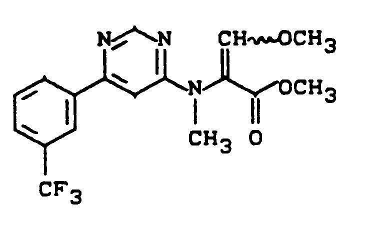

Ein Gemisch aus 7,0 g (21,4 mmol) [N-Methyl-N-[6-[(3-Trifluormethyl)phenyl]pyrimidin-4-yl]]amino-essigsäuremethylester, 100 ml Toluol und 4,63 ml einer 30%igen Natriummethanolat-Lösung in Methanol (25,8 mmol) werden unter 50 bar Kohlenmonoxid bei 70°C 5 Stunden zur Reaktion gebracht.

Man entspannt, kühlt auf 5°C ab und versetzt unter Rühren tropfenweise mit 5,3 g (42,0 mmol) Dimethylsulfat. Man rührt bei Raumtemperatur nach (ca. 12 Stunden), versetzt mit Wasser, extrahiert mit Ethylacetat, trocknet die organische Phase über wasserfreien Natriumsulfat und erhält nach dem Abziehen des Lösungsmittels im Vakuum einen Sirup, der säulenchromatographisch an Kieselgel gereinigt wird (Laufmittel: Dichlormethan/Ethylacetat 5:1).

Man erhält 80 % 3-Methoxy-2-[N-methyl-N- [6-[(Difluormethyl)phenyl]-pyrimidin-4-yl]amino-acrylsäuremethylester vom Schmelzpunkt 105 bis 108°C.

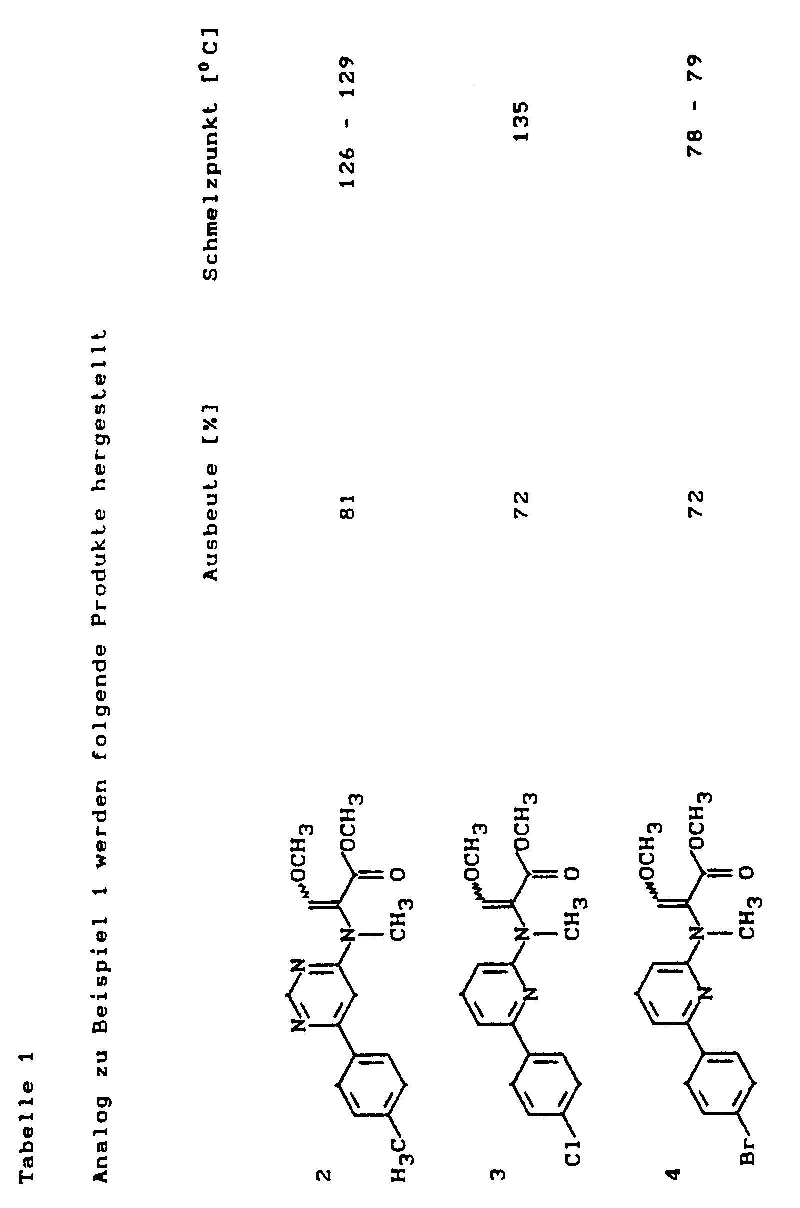

### Beispiel 5

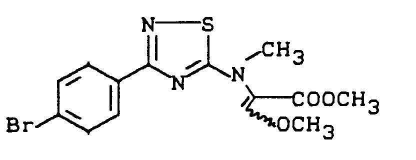

Ein Gemisch aus 16,8 g (49,1 mmol) [N-[3-(4-Brom-phenyl)-1,2,4-thiadiazol-5-yl]-N-methyl]amino-essigsäuremethylester, 3,2 g Natriummethylat-Pulver (92,7%ig, 54,9 mmol) und 100 ml Toluol wurde unter 50 bar Kohlenmonoxid bei 70°C bis zur Druckkonstanz zur Reaktion gebracht. Nach dem Entspannen wurde abgesaugt und der getrocknete Rückstand ¹H-NMR-spektroskopisch untersucht. Es handelte sich um das Natriumsalz des 2-[[N-[3-(4-Brom-phenyl)-1,2,4-thiadiazol-5-yl]-N-methyl]amino]-3-hydroxy-acrylsäuremethylesters (77,8 % d.Th.). Aus dem Filtrat konnten 18,3 % des Eduktes zurückgewonnen werden.

Das Natriumsalz wurde in 50 ml Dimethylformamid suspendiert und unter Rühren bei 0°C mit 8,5 g ( mmol) Dimethylsulfat versetzt. Man rührte noch ca. 2 Stunden bei Raumtemperatur nach und erhielt nach Aufarbeitung 13,8 g (73,1 %) 2-[[N-[3-(4-Brom-phenyl)-1,2,4-thiadiazol-5-yl]-N-methyl]amino]-3-methoxy-acrylsäuremethylester vom Schmelzpunk 91°C.

### Beispiel 6

Man verfährt analog Beispiel 5, wobei statt des Natriummethylat-Pulvers 10,7 g einer 30%igen Natriummethylatlösung in Methanol eingesetzt werden.

Die Ausbeute beträgt 93,2 %, es lassen sich 3,8 % Edukt wiedergewinnen.

### Vergleichsversuch A (vergleiche Herstellungsbeispiel 3)

Zu 2,4 g (0,1 Mol) Natriumhydrid in 60 ml Dimethylformamid gibt man tropfenweise unter Rühren bei Raumtemperatur 10,3 g (0,035 Mol) N-[6-(4-Chlorphenyl)-2-pyridyl]-N-methylaminoessigsäuremethylester und 50 g (1,2 Mol) Ameisensäuremethylester in 50 ml Dimethylformamid, rührt anschließend 3 Tage bei Raumtemperatur, gibt weitere 0,5 g (0,021 Mol) Natriumhydrid und 10 g (0,24 Mol) Ameisensäuremethylester zu, rührt weitere 5 Stunden bei Raumtemperatur, gibt danach abermals 0,5 g (0,021 Mol) Natriumhydrid und 10 g (0,24 Mol) Ameisensäuremethylester zu und rührt weitere 24 Stunden bei Raumtemperatur.

Danach gibt man 14,0 g (0,11 Mol) Dimethylsulfat zu und rührt weitere 48 Stunden bei Raumtemperatur. Zur Aufarbeitung gießt man die Reaktionsmischung in Wasser, extrahiert mit Ethylacetat, trocknet über Magnesiumsulfat, engt im Vakuum ein und chromatographiert den Rückstand an Kieselgel (Laufmittel: Dichlormethan/n-Hexan 5:1).

Man erhält als 1. Fraktion 7,1 g (69 % der Theorie) an Ausgangsverbindung N-[6-(4-Chlorphenyl)-2-pyridyl]-N-methylaminoessigsäuremethylester und als 2. Fraktion 0,5 g (4,2 % der Theorie) an gewünschtem Produkt 2-[N-[6-(4-Chlorphenyl)-2-pyridyl]-N-methylamino]-3-methoxyacrylsäuremethylester vom Schmelzpunkt 120°C.

### Vergleichsbeispiel B (vergleiche Herstellungsbeispiel 4)

Zu einem Gemisch aus 1,2 g (0,05 Mol) Natriumhydrid in 30 ml Dimethylformamid gibt man eine Lösung von 5,2 g (0,021 Mol) N-(6-Brom-2-pyridyl)-N-methylaminoessigsäuremethylester und 30 g (0,72 Mol) Ameisensäuremethylester in 30 ml Dimethylformamid, rührt anschließend 15 Stunden bei Raumtemperatur, gibt dann 12,5 g (0,1 Mol) Dimethylsulfat zu, rührt weitere 15 Stunden bei Raumtemperatur , gießt dann die Mischung in Wasser, extrahiert mit Ethylacetat, trocknet über Magnesiumsulfat, engt im Vakuum ein und chromatographiert den Rückstand an Kieselgel (Laufmittel: Dichlormethan/n-Hexan 5:1).

Man erhält als 1. Fraktion: 1,5 g (29 % der Theorie) an Ausgangsprodukt N-(6-Brom-2-pyridyl)-N-methylaminoessigsäuremethylester und als 2. Fraktion: 1,8 g (30 % der Theorie) an 2-[N-(6-Brom-2-pyridyl)-N-methylamino]-3-methoxyacrylsäuremethylester vom Schmelzpunkt 78 bis 79°C.

## Patentansprüche

1. Verfahren zur Herstellung von 3-Alkoxyacrylestern der allgemeinen Formel (I), 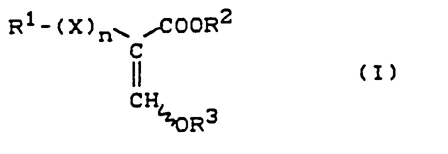 in welcher
R¹ für einen gegebenenfalls einfach oder mehrfach, gleich oder verschieden substituierten, gesättigten, einfach oder mehrfach ungesättigten oder aromatischen Carbocyclus mit 5 bis 10 Kohlenstoffatomen oder für einen gegebenenfalls einfach oder mehrfach, gleich oder verschieden substituierten, gesättigten, einfach oder mehrfach ungesättigten oder aromatischen Heterocyclus mit 2 bis 9 Kohlenstoffatomen und 1 bis 5 gleichen oder verschiedenen Heteroatomen - insbesondere Stickstoff, Sauerstoff oder Schwefel - steht, wobei als Substituenten jeweils infrage kommen:
Halogen, Cyano, Nitro, jeweils geradkettiges oder verzweigtes Alkyl, Alkoxy oder Alkylthio mit jeweils 1 bis 6 Kohlenstoffatomen, jeweils geradkettiges oder verzweigtes Halogenalkyl, Halogenalkoxy oder Halogenalkylthio mit jeweils 1 bis 4 Kohlenstoffatomen und 1 bis 9 gleichen oder verschiedenen Halogenatomen, jeweils geradkettiges oder verzweigtes Alkoxycarbonyl oder Alkoximinoalkyl, Dialkylamino oder Dialkylaminocarbonyl mit jeweils 1 bis 4 Kohlenstoffatomen in den einzelnen Alkylteilen, jeweils geradkettiges oder verzweigtes Alkenyl oder Alkinyl mit jeweils 2 bis 8 Kohlenstoffatomen, Cycloalkyl mit 3 bis 7 Kohlenstoffatomen, zweifach verknüpftes Alkandiyl mit 3 bis 5 Kohlenstoffatomen, oder jeweils im Arylteil oder im Heteroarylteil gegebenenfalls einfach oder mehrfach, gleich oder verschieden durch Halogen, Alkyl, Alkoxy, Alkylthio, Halogenalkyl, Halogenalkoxy, Halogenalkylthio, Dioxyalkylen, Halogen- substituiertes Dioxyalkylen oder gegebenenfalls substituiertes Phenyl substituiertes Aryl, Aryloxy, Arylthio, Arylcarbonyl, Aralkyl Aralkenyl, Aralkinyl, Aralkyloxy, Aralkylthio, Heteroarylalkyl, Heteroarylalkenyl, Heteroyryloxy, Heteroarylthio, Heteroarylcarbonyl, Heteroarylalkyloxy, Heteroarylalkylthio, oder Heteroaryl mit jeweils 6 bis 10 Kohlenstoffatomen im Arylteil oder mit 2 bis 9 Kohlenstoffatomen und 1 bis 4 gleichen oder verschiedenen Heteroatomen - insbesondere Stickstoff, Sauerstoff und/oder Schwefel - im Heteroarylteil und gegebenenfalls 1 bis 6 Kohlenstoffatomen im geradkettigen oder verzweigten Alkylteil oder gegebenenfalls 2 bis 6 Kohlenstoffatomen im geradkettigen oder verzweigten Alkenylteil beziehungsweise Alkinylteil;
R² für geradkettiges oder verzweigtes Alkyl mit 1 bis 6 Kohlenstoffatomen oder für gegebenenfalls im Arylteil einfach oder mehrfach, gleich oder verschieden substituiertes Aralkyl mit 1 bis 4 Kohlenstoffatomen im geradkettigen oder verzweigten Alkylteil und 6 bis 10 Kohlenstoffatomen im Arylteil steht, wobei als Arylsubstituenten infrage kommen: Halogen, Cyano, Nitro, jeweils geradkettiges oder verzweigtes Alkyl, Alkoxy oder Alkylthio mit jeweils 1 bis 4 Kohlenstoffatomen, jeweils geradkettiges oder verzweigtes Halogenalkyl, Halogenalkoxy oder Halogenalkylthio mit jeweils 1 bis 4 Kohlenstoffatomen und 1 bis 9 gleichen oder verschiedenen Halogenatomen, jeweils geradkettiges oder verzweigtes Alkoxycarbonyl oder Alkoximinoalkyl mit jeweils 1 bis 8 Kohlenstoffatomen in den einzelnen Alkylteilen, Cycloalkyl mit 3 bis 7 Kohlenstoffatomen, zweifach verknüpftes Alkandiyl mit 3 bis 5 Kohlenstoffatomen, jeweils gegebenenfalls im Arylteil einfach oder mehrfach, gleich oder verschieden durch Halogen, Alkyl, Alkoxy, Alkylthio, Halogenalkyl, Halogenalkoxy oder Halogenalkylthio mit jeweils 1 bis 4 Kohlenstoffatomen und gegebenenfalls 1 bis 9 gleichen oder verschiedenen Halogenatomen substituiertes Aryl, Aralkyl, Aryloxy oder Aralkyloxy mit jeweils 6 bis 10 Kohlenstoffatomen im Arylteil und gegebenenfalls 1 bis 4 Kohlenstoffatomen im geradkettigen oder verzweigten Alkylteil oder jeweils gegebenenfalls im Heteroarylteil einfach oder mehrfach, gleich oder verschieden durch Halogen, Alkyl, Alkoxy, Alkylthio, Halogenalkyl, Halogenalkoxy oder Halogenalkylthio mit jeweils 1 bis 4 Kohlenstoffatomen und gegebenenfalls 1 bis 9 gleichen oder verschiedenen Halogenatomen substituiertes Heteroarylalkyl oder Heteroaryl mit jeweils 2 bis 9 Kohlenstoffatomen und 1 bis 4 gleichen oder verschiedenen Heteroatomen, - insbesondere Stickstoff, Sauerstoff und/oder Schwefel - im Heteroarylteil und gegebenenfalls 1 bis 4 Kohlenstoffatomen im geradkettigen oder verzweigten Alkylteil;
R³ für geradkettiges oder verzweigtes Alkyl mit 1 bis 6 Kohlenstoffatomen oder für gegbenenfalls im Acrylteil einfach oder mehrfach, gleich oder verschieden substituiertes Aralkyl mit 1 bis 4 Kohlenstoffatomen im geradkettigen oder verzeigten Alkylteil und 6 bis 10 Kohlenstoffatomen im Arylteil steht, wobei als Arylsubstituenten infrage kommen: Halogen, Cyano, Nitro, jeweils geradkettiges oder verzweigtes Alkyl, Alkoxy oder Alkylthio mit jeweils 1 bis 4 Kohlenstoffatomen, jeweils geradkettiges oder verzweigtes Halogenalkyl, Halogenalkoxy oder Halogenalkylthio mit jeweils 1 bis 4 Kohlenstoffatomen und 1 bis 9 gleichen oder verschiedenen Halogenatomen, jeweils geradkettiges oder verzweigtes Alkoxycarbonyl oder Alkoximinoalkyl mit jeweils 1 bis 8 Kohlenstoffatomen in den einzelnen Alkylteilen, Cycloalkyl mit 3 bis 7 Kohlenstoffatomen, zweifach verknüpftes Alkandiyl mit 3 bis 5 Kohlenstoffatomen, jeweils gegebenenfalls im Arylteil einfach oder mehrfach, gleich oder verschieden durch Halogen, Alkyl, Alkoxy, Alkylthio, Halogenalkyl, Halogenalkoxy oder Halogenalkylthio mit jeweils 1 bis 4 Kohlenstoffatomen und gegebenenfalls 1 bis 9 gleichen oder verschiedenen Halogenatomen substituiertes Aryl, Aralkyl, Aryloxy oder Aralkyloxy mit jeweils 6 bis 10 Kohlenstoffatomen im Arylteil und gegebenenfalls 1 bis 4 Kohlenstoffatomen im geradkettigen oder verzweigten Alkylteil oder jeweils gegebenenfalls im Heteroarylteil einfach oder mehrfach, gleich oder verschieden durch Halogen, Alkyl, Alkoxy, Alkylthio, Halogenalkyl, Halogenalkoxy oder Halogenalkylthio mit jeweils 1 bis 4 Kohlenstoffatomen und gegebenenfalls 1 bis 9 gleichen oder verschiedenen Halogenatomen substitu-iertes Heteroarylalkyl oder Heteroaryl mit jeweils 2 bis 9 Kohlenstoffatomen und 1 bis 4 gleichen oder verschiedenen Heteroatomen, - insbesondere Stickstoff, Sauerstoff und/oder Schwefel - im Heteroarylteil und gegebenenfalls 1 bis 4 Kohlenstoffatomen im geradkettigen oder verzweigten Alkylteil;
X für Sauerstoff, Schwefel oder für einen der Reste 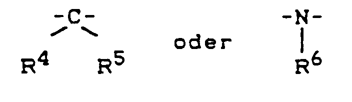 steht und
n für eine Zahl 0 oder 1 steht, wobei
R⁴, R⁵ und R⁶ unabhängig voneinander jeweils für Wasserstoff, für jeweils geradkettiges oder verzweigtes Alkyl mit 1 bis 6 Kohlenstoffatomen, Alkenyl mit 2 bis 6 Kohlenstoffatomen oder für jeweils gegebenenfalls im Arylteil einfach oder mehrfach, gleich oder verschieden substituiertes Aralkyl mit 1 bis 6 Kohlenstoffatomen im geradkettigen oder verzweigten Alkylteil und 6 bis 10 Kohlenstoffatomen im Arylteil oder Aralkenyl mit 2 bis 6 Kohlenstoffatomen im geradkettigen oder verzweigten Alkenylteil und 6 bis 10 Kohlenstoffatomen im Arylteil stehen, wobei als Arylsubstituenten jeweils die bei R² genannten infrage kommen,
dadurch gekennzeichnet, daß man substituierte Essigsäureester der Formel (II),
R¹-(X)ₙ-CH₂-COOR² (II)
in welcher
R¹, R², X und n die oben angegebene Bedeutung haben, in Gegenwart eines geeigneten Verdünnungsmittels zunächst mit Kohlenmonoxid bei einem Kohlenmonoxid-Partialdruck von 1 bis 200 bar und einem basischen Hilfsmittel umsetzt, und dabei Zwischenprodukte der allgemeinen Formel (III), 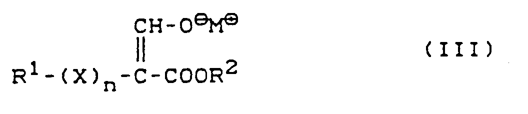 in welcher
R¹, R², X und n die oben angegebene Bedeutung haben
und
M^{⊕} für Wasserstoff oder das Äquivalent eines Alkalimetall- oder Erdalkalimetall- oder Ammoniumkations (vorzugsweise eines Natrium- oder Kaliumkations) steht,
erhält, wonach aus diesen Salzen
a) die freien Enole durch Ansäuern mit verdünnten Säuren und darauffolgende Extraktion mit einem geeigneten organischen Lösungsmittel isoliert werden und dann in einem geeigneten Verdünnungsmittel in Gegenwart einer geeigneten Base mit dem Alkylierungsmittel bei Temperaturen von 20° C bis 60° C alkyliert werden oder
b) die Enolatsalze der Formel (III) direkt im Reaktionsgemisch mit dem Alkylierungsmittel umgesetzt werden.

2. Verfahren gemäß Anspruch 1, dadurch gekennzeichnet, daß man die Umsetzung mit Kohlenmonoxid bei Temperaturen von 0 bis 200° C durchführt.

3. Verfahren gemäß Anspruch 1, dadurch gekennzeichnet, daß man die Umsetzung mit Kohlenmonoxid in Gegenwart eines Überschusses an basischem Hilfsmittel durchführt,

4. Verfahren gemäß Anspruch 1, dadurch gekennzeichnet, daß man die Umsetzung mit Kohlenmonoxid in Gegenwart einer stöchiometrischen Menge bis 35 % Überschuß an basischem Hilfsmittel durchführt.

## Claims

1. Process for the preparation of 3-alkoxyacrylates of the general formula (I) 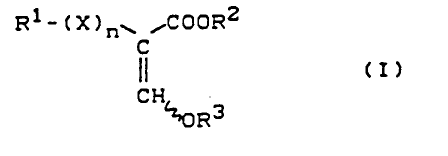 in which
R¹ represents a saturated, monounsaturated or polyunsaturated or aromatic carbocycle which has 5 to 10 carbon atoms and which is optionally monosubstituted or polysubstituted by identical or different substituents, or represents a saturated, monounsaturated or polyunsaturated or aromatic heterocycle which has 2 to 9 carbon atoms and 1 to 5 identical or different hetero atoms - in particular nitrogen, oxygen or sulphur - and which is optionally monosubstituted or polysubstituted by identical or different substituents, suitable substituents in each case being:
halogen, cyano, nitro, in each case straight-chain or branched alkyl, alkoxy or alkylthio, each of which has 1 to 6 carbon atoms, in each case straight-chain or branched halogenoalkyl, halogenoalkoxy or halogenoalkylthio, each of which has 1 to 4 carbon atoms and 1 to 9 identical or different halogen atoms, in each case straight-chain or branched alkoxycarbonyl or alkoximinoalkyl, dialkylamino or dialkylaminocarbonyl, each of which has 1 to 4 carbon atoms in the individual alkyl moieties, in each case straight-chain or branched alkenyl or alkinyl, each of which has 2 to 8 carbon atoms, cycloalkyl having 3 to 7 carbon atoms, divalent alkanediyl having 3 to 5 carbon atoms, or aryl, aryloxy, arylthio, arylcarbonyl, aralkyl aralkenyl, aralkinyl, aralkyloxy, aralkylthio, heteroarylalkyl, heteroarylalkenyl, heteroyryloxy, heteroarylthio, heteroarylcarbonyl, heteroarylalkyloxy, heteroarylalkylthio, or heteroaryl, each of which has 6 to 10 carbon atoms in the aryl moiety or 2 to 9 carbon atoms and 1 to 4 identical or different hetero atoms - in particular nitrogen, oxygen and/or sulphur - in the heteroaryl moiety and, if appropriate, 1 to 6 carbon atoms in the straight-chain or branched alkyl moiety or, if appropriate, 2 to 6 carbon atoms in the straight-chain or branched alkenyl moiety, or alkinyl moiety, respectively, and each of which is optionally monosubstituted or polysubstituted in the aryl moiety or in the heteroaryl moiety by identical or different substituents from the series comprising halogen, alkyl, alkoxy, alkylthio, halogenoalkyl, halogenoalkoxy, halogenoalkylthio, dioxyalkylene, halogen-substituted dioxyalkylene or optionally substituted phenyl;
R² represents straight-chain or branched alkyl having 1 to 6 carbon atoms, or represents aralkyl which has 1 to 4 carbon atoms in the straight-chain or branched alkyl moiety and 6 to 10 carbon atoms in the aryl moiety and which is optionally monosubstituted or polysubstituted in the aryl moiety by identical or different substituents, suitable aryl substituents being:
halogen, cyano, nitro, in each case straight-chain or branched alkyl, alkoxy or alkylthio, each of which has 1 to 4 carbon atoms, in each case straight-chain or branched halogenoalkyl, halogenoalkoxy or halogenoalkylthio, each of which has 1 to 4 carbon atoms and 1 to 9 identical or different halogen atoms, in each case straight-chain or branched alkoxycarbonyl or alkoximinoalkyl, each of which has 1 to 8 carbon atoms in the individual alkyl moieties, cycloalkyl having 3 to 7 carbon atoms, divalent alkanediyl having 3 to 5 carbon atoms, aryl, aralkyl, aryloxy or aralkyloxy, each of which has 6 to 10 carbon atoms in the aryl moiety and, if appropriate, 1 to 4 carbon atoms in the straight-chain or branched alkyl moiety and each of which is optionally monosubstituted or polysubstituted in the aryl moiety by identical or different substituents from the series comprising halogen, alkyl, alkoxy, alkylthio, halogenoalkyl, halogenoalkoxy or halogenoalkylthio, each of which has 1 to 4 carbon atoms and, if appropriate, 1 to 9 identical or different halogen atoms, or heteroarylalkyl or heteroaryl, each of which has 2 to 9 carbon atoms and 1 to 4 identical or different hetero atoms, - in particular nitrogen, oxygen and/or sulphur - in the heteroaryl moiety and, if appropriate, 1 to 4 carbon atoms in the straight-chain or branched alkyl moiety and each of which is optionally monosubstituted or polysubstituted in the heteroaryl moiety by identical or different substituents from the series comprising halogen, alkyl, alkoxy, alkylthio, halogenoalkyl, halogenoalkoxy or halogenoalkylthio, each of which has 1 to 4 carbon atoms and, if appropriate, 1 to 9 identical or different halogen atoms;
R³ represents straight-chain or branched alkyl having 1 to 6 carbon atoms, or represents aralkyl which has 1 to 4 carbon atoms in the straight-chain or branched alkyl moiety and 6 to 10 carbon atoms in the aryl moiety and which is optionally monosubstituted or polysubstituted in the acryl moiety by identical or different substituents, suitable aryl substituents being: halogen, cyano, nitro, in each case straight-chain or branched alkyl, alkoxy or alkylthio, each of which has 1 to 4 carbon atoms, in each case straight-chain or branched halogenoalkyl, halogenoalkoxy or halogenoalkylthio, each of which has 1 to 4 carbon atoms and 1 to 9 identical or different halogen atoms, in each case straight-chain or branched alkoxycarbonyl or alkoximinoalkyl, each of which has 1 to 8 carbon atoms in the individual alkyl moieties, cycloalkyl having 3 to 7 carbon atoms, divalent alkanediyl having 3 to 5 carbon atoms, aryl, aralkyl, aryloxy or aralkyloxy, each of which has 6 to 10 carbon atoms in the aryl moiety and, if appropriate, 1 to 4 carbon atoms in the straight-chain or branched alkyl moiety and each of which is optionally monosubstituted or polysubstituted in the aryl moiety by identical or different substituents from the series comprising halogen, alkyl, alkoxy, alkylthio, halogenoalkyl, halogenoalkoxy or halogenoalkylthio, each of which has 1 to 4 carbon atoms and, if appropriate, 1 to 9 identical or different halogen atoms, or heteroarylalkyl or heteroaryl, each of which has 2 to 9 carbon atoms and 1 to 4 identical or different hetero atoms, - in particular nitrogen, oxygen and/or sulphur - in the heteroaryl moiety and, if appropriate, 1 to 4 carbon atoms in the straight-chain or branched alkyl moiety and each of which is optionally monosubstituted or polysubstituted in the heteroaryl moiety by identical or different substituents from the series comprising halogen, alkyl, alkoxy, alkylthio, halogenoalkyl, halogenoalkoxy or halogenoalkylthio, each of which has 1 to 4 carbon atoms and, if appropriate, 1 to 9 identical or different halogen atoms;
X represents oxygen, sulphur or one of the radicals 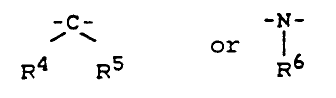 and
n represents a number 0 or 1, where
R⁴, R⁵ and R⁶ independently of one another in each case represent hydrogen, in each case straight-chain or branched alkyl having 1 to 6 carbon atoms, alkenyl having 2 to 6 carbon atoms, or aralkyl which has 1 to 6 carbon atoms in the straight-chain or branched alkyl moiety and 6 to 10 carbon atoms in the aryl moiety or aralkenyl which has 2 to 6 carbon atoms in the straight-chain or branched alkenyl moiety and 6 to 10 carbon atoms in the aryl moiety, each of which is optionally monosubstituted or polysubstituted in the aryl moiety by identical or different substituents, suitable aryl substituents in each case being those mentioned in the case of R²,
characterised in that substituted acetates of the formula (II)
R¹-(X)ₙ-CH₂-COOR² (II)
in which
R¹, R², X and n have the abovementioned meaning are first reacted with carbon monoxide at a carbon monoxide partial pressure of 1 to 200 bar and a basic auxiliary in the presence of a suitable diluent, and intermediates of the general formula (III) 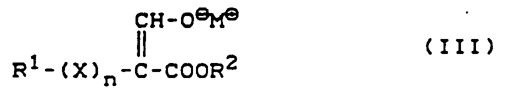 in which
R¹, R², X and n have the abovementioned meaning and
M^{⊕} represents hydrogen or the equivalent of an alkali metal cation or alkaline earth metal cation or ammonium cation (preferably of a sodium or potassium cation),
are obtained, whereupon from these salts
a) the free enols are isolated by acidification with dilute acids followed by extraction with a suitable organic solvent, and are then alkylated with the alkylating agent in a suitable diluent in the presence of a suitable base at temperatures from 20°C to 60°C, or
b) the enolate salts of the formula (III) are reacted with the alkylating agent directly in the reaction mixture.

2. Process according to Claim 1, characterised in that the reaction is carried out with carbon monoxide at temperatures from 0 to 200°C.

3. Process according to Claim 1, characterised in that the reaction is carried out with carbon monoxide in the presence of an excess of basic auxiliary.

4. Process according to Claim 1, characterised in that the reaction is carried out with carbon monoxide in the presence of a stoichiometric amount ups to an excess of 35% of basic auxiliary.

## Revendications

1. Procédé de production d'esters 3-alkoxy-acryliques de formule générale (I), 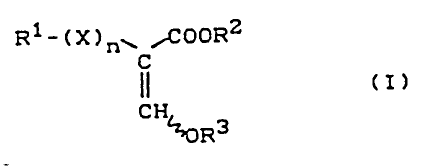 dans laquelle
R¹ représente un carbocycle de 5 à 10 atomes de carbone saturés, mono-insaturés ou polyinsaturés ou aromatiques, portant, le cas échéant, un ou plusieurs substituants identiques ou différents ou un hétérocycle saturé, monoinsaturé ou poly-insaturé ou aromatique portant, le cas échéant, un ou plusieurs substituants identiques ou différents et ayant 2 à 9 atomes de carbone et 1 à 5 hétéroatomes - notamment azote, oxygène ou soufre - identiques ou différents, avec dans chaque cas comme substituants :
un halogène, un groupe cyano, un groupe nitro, un groupe alkyle, alkoxy ou alkylthio ayant chacun 1 à 6 atomes de carbone et étant chacun linéaire ou ramifié, un groupe halogénalkyle, halogénalkoxy ou halogénalkylthio ayant chacun 1 à 4 atomes de carbone et 1 à 9 atomes d'halogènes identiques ou différents et étant chacun linéaire ou ramifié, un groupe alkoxycarbonyle ou alkoximinoalkyle, dialkylamino ou dialkylaminocarbonyle ayant chacun 1 à 4 atomes de carbone dans les parties alkyle individuelles et étant chacun linéaire ou ramifié, un groupe alcényle ou alcynyle ayant chacun 2 à 8 atomes de carbone et étant chacun linéaire ou ramifié, un groupe cycloalkyle ayant 3 à 7 atomes de carbone, un groupe alcanediyle divalent ayant 3 à 5 atomes de carbone, ou un groupe aryle, aryloxy, arylthio, arylcarbonyle, aralkyle, aralcényle, aralcynyle, aralkyloxy, aralkylthio et hétéroarylalkyle, hétéroarylalcényle, hétéroaryloxy, hétéroarylthio, hétéroarylcarbonyle, hétéroarylalkyloxy, hétéroarylalkylthio ou hétéroaryle portant chacun, le cas échéant, dans la partie aryle ou dans la partie hétéroaryle, un ou plusieurs substituants, identiques ou différents, halogéno, alkyle, alkoxy, alkylthio, halogénalkyle, halogénalkoxy, halogénalkylthio, dioxyalkylène, dioxyalkylène substitué par un halogène ou phényle éventuellement substitué, ayant chacun 6 à 10 atomes de carbone dans la partie aryle ou 2 à 9 atomes de carbone et 1 à 4 hétéroatomes - notamment azote, oxygène et/ou soufre - identiques ou différents dans la partie hétéroaryle et, le cas échéant, 1 à 6 atomes de carbone dans la partie alkyle linéaire ou ramifiée ou, le cas échéant, 2 à 6 atomes de carbone dans la partie alcényle ou la partie alcynyle linéaire ou ramifiée ;
R² désigne un groupe alkyle linéaire ou ramifié ayant 1 à 6 atomes de carbone ou un groupe aralkyle portant, le cas échéant dans la partie aryle, un ou plusieurs substituants identiques ou différents et ayant 1 à 4 atomes de carbone dans la partie alkyle linéaire ou ramifiée et 6 à 10 atomes de carbone dans la partie aryle, avec comme substituants de la partie aryle : un halogène, un groupe cyano, nitro, un groupe alkyle, alkoxy ou alkylthio étant chacun linéaire ou ramifié et ayant chacun 1 à 4 atomes de carbone, un groupe halogénalkyle, halogénalkoxy ou halogénalkylthio étant chacun linéaire ou ramifié et ayant chacun 1 à 4 atomes de carbone et 1 à 9 atomes d'halogènes identiques ou différents, un groupe alkoxycarbonyle ou alkoximinoalkyle étant chacun linéaire ou ramifié et ayant chacun 1 à 8 atomes de carbone dans les parties alkyle individuelles, un groupe cycloalkyle ayant 3 à 7 atomes de carbone, un groupe alcanediyle divalent ayant 3 à 5 atomes de carbone, un groupe aryle, aralkyle, aryloxy ou aralkyloxy portant chacun, le cas échéant dans la partie aryle, un ou plusieurs substituants, identiques ou différents, halogéno, alkyle, alkoxy, alkylthio, halogénalkyle, halogénalkoxy ou halogénalkylthio ayant chacun 1 à 4 atomes de carbone et, le cas échéant, 1 à 9 atomes d'halogènes identiques ou différents, ayant chacun 6 à 10 atomes de carbone dans la partie aryle et, le cas échéant, 1 à 4 atomes de carbone dans la partie alkyle linéaire ou ramifiée, ou un groupe hétéroarylalkyle ou hétéroaryle portant chacun, le cas échéant dans la partie hétéroaryle, un ou plusieurs substituants, identiques ou différents, halogéno, alkyle, alkoxy, alkylthio, halogénalkyle, halogénalkoxy ou halogénalkylthio ayant chacun 1 à 4 atomes de carbone et, le cas échéant, 1 à 9 atomes d'halogènes identiques ou différents, ayant dans chaque cas 2 à 9 atomes de carbone et 1 à 4 hétéroatomes - notamment azote, oxygène et/ou soufre - identiques ou différents dans la partie hétéroaryle et, le cas échéant, 1 à 4 atomes de carbone dans la partie alkyle linéaire ou ramifiée ;
R³ représente un groupe alkyle linéaire ou ramifié ayant 1 à 6 atomes de carbone ou un groupe aralkyle ayant 1 à 4 atomes de carbone dans la partie alkyle linéaire ou ramifiée et 6 à 10 atomes de carbone dans la partie aryle, portant, le cas échéant dans la partie aryle, un ou plusieurs substituants identiques ou différents, avec comme substituants de la partie aryle : un halogène, un groupe cyano, nitro, un groupe alkyle, alkoxy ou alkylthio étant chacun linéaire ou ramifié et ayant chacun 1 à 4 atomes de carbone, un groupe halogénalkyle, halogénalkoxy ou halogénalkylthio étant chacun linéaire ou ramifié et ayant chacun 1 à 4 atomes de carbone et 1 à 9 atomes d'halogènes identiques ou différents, un groupe alkoxycarbonyle ou alkoximinoalkyle étant chacun linéaire ou ramifié et ayant chacun 1 à 8 atomes de carbone dans les parties alkyle individuelles, un groupe cycloalkyle ayant 3 à 7 atomes de carbone, un groupe alcanediyle divalent ayant 3 à 5 atomes de carbone, un groupe aryle, aralkyle, aryloxy ou aralkoxy portant chacun, le cas échéant dans la partie aryle, un ou plusieurs substituants, identiques ou différents, halogéno, alkyle, alkoxy, alkylthio, halogénalkyle, halogénalkoxy ou halogénalkylthio ayant chacun 1 à 4 atomes de carbone et, le cas échéant, 1 à 9 atomes d'halogènes identiques ou différents, ayant dans chaque cas 6 à 10 atomes de carbone dans la partie aryle et, le cas échéant, 1 à 4 atomes de carbone dans la partie alkyle linéaire ou ramifiée ou un groupe hétéroarylalkyle ou hétéroaryle ayant chacun 2 à 9 atomes de carbone et 1 à 4 hétéroatomes - notamment azote, oxygène et/ou soufre - identiques ou différents dans la partie hétéroaryle et, le cas échéant, 1 à 4 atomes de carbone dans la partie alkyle linéaire ou ramifiée et portant chacun, le cas échéant dans la partie hétéroaryle, un ou plusieurs substituants, identiques ou différents, halogéno, alkyle, alkoxy, alkylthio, halogénalkyle, halogénalkoxy ou halogénalkylthio ayant chacun 1 à 4 atomes de carbone et, le cas échéant, 1 à 9 atomes d'halogènes identiques ou différents ;
X représente de l'oxygène, du soufre ou l'un des restes 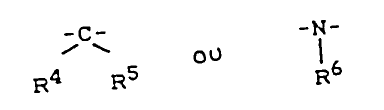 et
n représente le nombre 0 ou 1,
R⁴, R⁵ et R⁶ représentant chacun indépendamment des autres un atome d'hydrogène, un groupe alkyle linéaire ou ramifié ayant 1 à 6 atomes de carbone, alcényle linéaire ou ramifié ayant 2 à 6 atomes de carbone ou un groupe aralkyle ayant 1 à 6 atomes de carbone dans la partie alkyle linéaire ou ramifiée et 6 à 10 atomes de carbone dans la partie aryle ou un groupe aralcényle ayant 2 à 6 atomes de carbone dans la partie alcényle linéaire ou ramifiée et 6 à 10 atomes de carbone dans la partie aryle, chacun portant, le cas échéant dans la partie aryle, un ou plusieurs substituants identiques ou différents, avec dans chaque cas comme substituants de la partie aryle les substituants mentionnés pour R²,
caractérisé en ce qu'on fait réagir des esters d'acide acétique substitué de formule (II),
R¹-(X)ₙ-CH₂-COOR² (II)
dans laquelle
R¹, R², X et n ont la définition indiquée ci-dessus, en présence d'un diluant approprié, tout d'abord avec du monoxyde de carbone sous pression partielle de monoxyde de carbone de 1 à 200 bars et avec un agent auxiliaire basique, et on obtient alors des produits intermédiaires de formule générale (III), 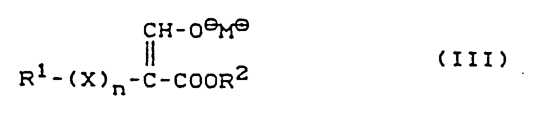 dans laquelle
R¹, R², X et n ont la définition indiquée ci-dessus et
M^{⊕} représente de l'hydrogène ou l'équivalent d'un cation de métal alcalin ou de métal alcalinoterreux ou d'ammonium (de préférence un cation sodium ou potassium),
puis on isole de ces sels
a) les énols libres par acidification avec des acides dilués suivie d'une extraction avec un solvant organique approprié et on les alkyle ensuite dans un diluant approprié en présence d'une base appropriée avec l'agent d'alkylation à des températures de 20°C à 60°C, ou bien
b) on fait réagir les énolates-sels de formule (III) directement dans le mélange réactionnel avec l'agent d'alkylation.

2. Procédé suivant la revendication 1, caractérisé en ce qu'on conduit la réaction avec le monoxyde de carbone à des températures de 0 à 200°C.

3. Procédé suivant la revendication 1, caractérisé en ce qu'on conduit la réaction avec le monoxyde de carbone en présence d'un excès d'agent auxiliaire basique.

4. Procédé suivant la revendication 1, caractérisé en ce qu'on conduit la réaction avec le monoxyde de carbone en présence d'une quantité d'agent auxiliaire basique allant de la stoechiométrie jusqu'à un excès de 35 %.
